# EUROPÄISCHE PATENTANMELDUNG

(11) **EP 3 904 327 A1**
(43) Veröffentlichungstag der Anmeldung: **03.11.2021**
(21) Anmeldenummer: 20172256.8
(22) Anmeldetag: 30.04.2020
(51) Int. Cl.: C07C 67/39, C07C 69/54

(54) **VERFAHREN ZUR HERSTELLUNG VON HYDROXYALKYL(METH)ACRYLSÄUREESTERN DURCH OXIDATIVE SPALTUNG VON METHACROLEIN-ACETALEN**

(71) Anmelder: Röhm GmbH, 64295 Darmstadt (DE)
(72) Erfinder: Die Erfindernennung liegt noch nicht vor
(74) Vertreter: Röhm Patent Association

(57) **Zusammenfassung**

Die vorliegende Erfindung betrifft ein Verfahren zur Herstellung von Hydroxyalkyl(meth)acrylestern, insbesondere Hydroxyethylmethacrylat (HEMA), umfassend in einer ersten Reaktionsstufe die Umsetzung von (Meth)acrolein mit mindestens einem mehrwertigen Alkohol, insbesondere Ethylenglykol, in Gegenwart eines ersten Katalysators K1, wobei ein erstes Reaktionsprodukt enthaltend ein zyklisches Acetal erhalten wird, und in einer zweiten Reaktionsstufe die Umsetzung des ersten Reaktionsproduktes mit Sauerstoff in Gegenwart eines zweiten Katalysators K2, wobei ein zweites Reaktionsprodukt, enthaltend mindestens einen Hydroxyalkyl(meth)acrylester, erhalten wird, wobei nach der ersten Reaktionsstufe Wasser und optional weitere Komponenten, insbesondere (Meth)acrolein und/oder der mehrwertige Alkohol, z.B. Ethylenglykol, zumindest teilweise aus dem ersten Reaktionsprodukt entfernt werden.

## Beschreibung

Die vorliegende Erfindung betrifft ein Verfahren zur Herstellung von Hydroxyalkyl(meth)acrylestern, insbesondere Hydroxyethylmethacrylat (HEMA), umfassend in einer ersten Reaktionsstufe die Umsetzung von (Meth)acrolein mit mindestens einem mehrwertigen Alkohol, insbesondere Ethylenglykol, in Gegenwart eines ersten Katalysators K1, wobei ein erstes Reaktionsprodukt enthaltend ein zyklisches Acetal erhalten wird, und in einer zweiten Reaktionsstufe die Umsetzung des ersten Reaktionsproduktes mit Sauerstoff in Gegenwart eines zweiten Katalysators K2, wobei ein zweites Reaktionsprodukt, enthaltend mindestens einen Hydroxyalkyl(meth)acrylester, erhalten wird, wobei nach der ersten Reaktionsstufe Wasser und optional weitere Komponenten, insbesondere (Meth)acrolein und/oder der mehrwertige Alkohol, z.B. Ethylenglykol, zumindest teilweise aus dem ersten Reaktionsprodukt entfernt werden.

Hydroxyalkylester auf Basis von Methacrylsäure und/oder Acrylsäure stellen technisch wichtige Monomere oder Comonomere für die Herstellung von Polymethyl(meth)acrylaten dar. Hydroxyalkyl(meth)acrylester und daraus hergestellte Polymere werden für verschiedene Anwendungen eingesetzt, beispielsweise für Lacke, Haftmittel, Kontaktlinsen, Polymervernetzer und Materialien für den 3D-Druck. Insbesondere Hydroxyethylmethacrylat (HEMA) ist von technischer Bedeutung.

### Stand der Technik

Im Stand der Technik wird oftmals die Herstellung von HEMA ausgehend von Methacrylsäure und Ethylenoxid unter Verwendung von Chrom-Katalysatoren beschrieben, z.B. WO 2012/116870 A1, JP 5 089 964 B2 und US 2015/01267670. Oftmals wird dem sehr reaktionsfähigen Hydroxyalkyl(meth)acrylester-Monomer ein Stabilisator zugesetzt (z.B. EP-B 1 125 919).

Neben der Herstellung von HEMA ausgehend von Methacrylsäure wird im Stand der Technik auch die Herstellung von anderen Hydroxyalkyl(meth)acrylestern beschrieben, wobei beispielsweise Propylenoxid oder andere substituierte Oxirane eingesetzt werden, z.B. JP 2008143814, JP 2008127302. Üblicherweise wird die Umsetzung von (Meth)acrylsäure mit den entsprechenden Oxiranen mit homogenen Katalysatoren durchgeführt, wobei die (Meth)acrylsäure, anteilig oder vollständig in Gegenwart des gelösten Katalysators sowie einem Stabilisator, vorgelegt wird und das Oxiran gasförmig oder flüssig zu dosiert wird. Neben der Dosierung, ist auch die Durchmischung der Reaktanden und des Katalysators von Bedeutung, damit der gewünschte Hydroxyalkyl(meth)acrylester in diesen Reaktionen in hoher, konstanter Produktqualität erhalten wird, und damit die Anlage im Dauerbetrieb genutzt und leicht gereinigt werden kann. Eine hierfür geeignete Ausführungsform ist beispielsweise in WO 2012/1168770 A1 beschrieben, wobei der Reaktor mit einer Injektor-Mischdüse und einer Umwälzleitung ausgestattet ist. Gegen Reaktionsende liegt typischerweise der gewünschte Hydroxyalkyl(meth)acrylestern in hoher Konzentration vor, während der Ausgangsstoff Methacrylsäure stark reduziert oder nicht mehr vorhanden ist.

Die anschließende Aufarbeitung und Isolierung der Hydroxyalkyl(meth)acrylester erfolgt oftmals destillativ und kann kontinuierlich betrieben werden (z.B. beschrieben in DE 10 2007 056926 A1 und EP 1090904 A2).

Die destillative Aufreinigung erfolgt für kommerzielle Produkte in der Regel bis zu einer Reinheit größer 97%, wobei für Spezialanwendungen die Nebenkomponenten auf Säurebasis und Vernetzer weiter reduziert sind. Der Gehalt an Hydroxyalkyl(meth)acrylester beträgt dabei üblicherweise mehr als 99%. Eine solche Aufreinigung erfolgt in mehreren Destillationsschritten und ist beispielsweise in der EP 2427421 B1 beschrieben. Unabhängig von der gewünschten Produktqualität muss oftmals während der Aufreinigung mindestens ein Stabilisator zugegeben werden. Das spezifikationsgerechte Produkt kann beispielsweise Hydrochinonmonomethylether und 4-Hydroxy-2,2,6,6-tetramethylpiperidin-N-oxyl (TEMPOL) oder auch Kombinationen von mehreren Stabilisatoren enthalten (z.B. in der WO 2010/105894 A2 beschrieben). Es sind auch Produktzusammensetzungen beschrieben, die neben Stabilisatoren auch Nebenprodukte der Reaktion, wie Hydroxyethylacetat, enthalten, und die eine besondere Lagerstabilität aufweisen sollen (z.B. in der EP 1125919 A2 beschrieben).

Die in der Reaktion eingesetzten Katalysatoren basieren häufig auf Chromsalzen, wie z.B. in den Schriften WO 2012/116870 A1, JP 5 089 964 B2 und US 2015/01267670 aufgezeigt. Neben Chrom-Katalysatoren werden auch Katalysatoren auf Basis von Eisensalzen, wie z.B. in US 4,365,081, beschrieben. Eine Vielzahl anderer metallhaltiger, löslicher Katalysatoren aus der Gruppe der Übergangsmetalle mit verschiedensten Gegenionen bzw. Liganden ist in der Literatur beschrieben.

Problematisch bei diesen bekannten und gängigen Herstellverfahren ist die Verwendung von Ethylenoxid, das eine sehr giftige, krebserregende, hochentzündliche und explosive Verbindung darstellt. Folglich stellen diese Verfahren sehr große Anforderungen an Handhabung, Transport, Lagerung etc. von Ethylenoxid. Der Transport und die Verwendung von Ethylenoxid sind in der EU und anderen Ländern, z.B. USA, stark reglementiert und daher aufwändig. Informationen, die USA betreffend, werden beispielsweise im EPA Clean Air Act beschrieben. Eine Verschärfung der Auflagen für den Umgang mit Ethylenoxid in den USA ist auf Basis der in 2020 anstehenden Aktualisierung der "National Emission Standards for Hazardous Air Pollutants (NESHAP)" wahrscheinlich.

Der Ausbau von Kapazitäten zur Herstellung von HEMA ist daher auf Grund der lokalen Verfügbarkeit sowie Regulation von Ethylenoxid (z.B. EPA Clean Air Act für USA) oftmals begrenzt.

In den bekannten Verfahren ist die Verwendung von Ethylenoxid insbesondere dann kritisch zu beurteilen, wenn Ethylenoxid im Überschuss bzw. stationär in vergleichsweise hohen Konzentrationen vorliegt. In den meisten im Stand der Technik beschriebenen Verfahren ist dies der Fall. Somit müssen erhöhte sicherheitstechnische Vorkehrungen getroffen werden, insbesondere müssen Einmischen und stationäre Konzentration von Ethylenoxid über das Einstellen von Dosierzeiten und Dosierraten sowie die Temperaturkontrolle und Abführung der Reaktionsexothermie streng reguliert werden.

Ein weiterer Nachteil der Herstellverfahren des Standes der Technik ist die Verwendung von giftigen und krebserregenden Chromsalzen, oftmals in der Oxidationsstufe +VI. Erste Chromsalze anderer Oxidationsstufen sind bezüglich Anwendung und Entsorgung bereits kritisch eingestuft (siehe beispielsweise Aktenzeichen WD5-3000-044/19 des deutschen Bundestages sowie die REACH Annex XVII zu Chrom +VI Verbindungen). Es ist zu erwarten, dass weitere Verbote, Vorgaben und Regularien zur verschärften Arbeitssicherheit in Zukunft folgen, was zumindest einen Mehraufwand zur Folge hat. Rückstande der Chromsalze fallen bei allen etablierten Verfahren, insbesondere bei der Aufarbeitung als Sumpfrückstand der Destillation, an. Dieser Sumpfrückstand muss aufwändig und sorgfältig entsorgt werden, um sicherzustellen, dass kein Chrom in die Umwelt gelangt. Damit ergeben sich zusammenfassend Nachteile der etablierten Verfahren bezüglich der hohen Kosten für den Einsatz der Katalysatoren und der resultierenden Entsorgung der Katalysatorrückstände, sowie hoher Investitionskosten der Produktionsanlage und hohe, laufende Betriebskosten durch lange Reaktionszeiten.

EP 0 704 441 A2 beschreibt ein Verfahren zur Herstellung von 2-Vinyl-1,3-dioxolan durch Umsetzung von Acrolein mit Ethylenglykol in Gegenwart eines festen sauren Katalysators. Hier wird zudem darauf hingewiesen, dass die Reaktion bei niedrigen Temperaturen von unter 20 °C eine höhere Selektivität aufweist. Es wird beschrieben, dass sich bei erhöhter Temperatur vermehrt Nebenprodukte bilden durch die Addition des Alkohols an die 4-Position von Acrolein im Sinne einer Michael-artigen Reaktion. Bei der Verwendung von mehrwertigen Alkoholen ist zudem zu erwarten, dass oligomere und polymere Verbindungen gebildet werden, was sowohl die Reaktionsführung, als auch die Aufreinigung erschweren würde. Hierzu macht diese Veröffentlichung keine klaren Aussagen.

Die EP 0 485 785 A1 beschreibt ein Verfahren zur Herstellung von alpha, beta-ungesättigten Acetalen, insbesondere ausgehend von Methacrolein, wobei als Alkohol Methanol eingesetzt wird. Dabei werden Methanol und Methacrolein destillativ vom Acetal abgetrennt und bei Raumtemperatur zur Reaktion gebracht. Nicht umgesetztes Startmaterial läuft zusammen mit dem Acetal in die Sumpfvorlage der Kolonne. Das hier beschriebene Verfahren ist jedoch nicht anwendbar für zweiwertige Alkohole, wie Ethylenglykol, da der zweiwertige Alkohol einen höheren Siedepunkt als das korrespondierende Dioxolan aufweist. Entsprechend sind für eine solche Reaktion große Mengen an Nebenprodukten analog der Beschreibung der EP 0 704 441 A2 zu erwarten.

Das Dokument JPH11315075A beschreibt die Umsetzung von Methacrolein mit Ethylenglykol unter Einsatz eines heterogenen Katalysators z.B. eines Zeoliths oder Mischungen aus Siliziumdioxid und Aluminiumoxid und eines azeotropen Schleppmittels, wie Cyclohexan oder Toluol. Hierbei werden nur Batchprozesse beschrieben, die aufgrund der Natur der gewählten Schleppmittel bei hohen Temperaturen betrieben werden müssen.

Die japanische Patentanmeldung JP 43-11205 beschreibt die oxidative Spaltung eines zyklischen Acetals, um einen ungesättigten Hydroxyester auf Basis von Acrylat zu erhalten. Ebenfalls wird die oxidative Spaltung eines zyklischen Acetals auf Basis von Methacrolein beschrieben, wodurch 2-Hydroxyethylmethacrylat erhalten wird. Jedoch wird hier innerhalb von 6 Stunden ein Umsatz von nur 25% erhalten, was bezogen auf die Raum-Zeit-Ausbeute als ungenügend und wenig wirtschaftlich anzusehen ist. Die Herstellung der zyklischen Acetale wird in dem Dokument nicht beschrieben.

Die japanische Patentanmeldung JP 2009 274987 beschreibt ein Verfahren zur Herstellung von Hydroxyalkyl(meth)acrylaten, z.B. Hydroxyethyl(meth)acrylat oder Hydroxypropyl(meth)acrylat, durch Oxidation eines zyklischen Acetals in Gegenwart eines speziellen heterogenen Edelmetall-haltigen Katalysators. Die hier beschriebenen Verfahren sind jedoch, insbesondere auf Grund der langen Reaktionszeiten, für die großtechnische Anwendung, insbesondere in einem kontinuierlichen Verfahren, ungeeignet.

Die chemoselektive Oxidation von aromatischen Acetalen durch Sauerstoff unter Bildung von Hydroxyalkylestern unter Verwendung eines Palladium-Katalysators ist zudem beschrieben in Sawama et al. (Org. Lett. 2016, 18, 5604). Die Umsetzung nicht-aromatischer Substrate und insbesondere von alpha,beta-ungesättigten Verbindungen, wird hier nicht beschrieben. Gemäß Sawama et al. wird Methanol oder Ethylenglykol als bestes Lösungsmittel beschrieben.

In dem Dokument US 9 593 064 B2 (S.S. Stahl et al.) werden Palladium-Katalysatoren auf Aktivkohle beschrieben, die durch zwei Dopanten, beispielsweise Bismuth und Tellur, ergänzt werden. Die Katalysatoren werden zur Herstellung von Estern durch direkte oxidative Veresterung von organischen Alkoholen in der Gegenwart von Methanol oder Ethanol eingesetzt. Zyklische Acetale werden als Substrate nicht beschrieben.

In Anbetracht des Standes der Technik ist es daher Aufgabe der vorliegenden Erfindung, ein Verfahren zur Herstellung von Hydroxyalkyl(meth)acrylestern, insbesondere Hydroxyethylmethacrylat (HEMA), bereitzustellen, wobei Ethylenoxid und gegebenenfalls auch Methacrylsäure, durch andere Edukte ersetzt sind, welche sicher, preiswert und global gut verfügbar sind. Zudem soll das Herstellverfahren insbesondere konkurrenzfähig sein zu bekannten Verfahren des Standes der Technik und nicht mit den oben beschriebenen Nachteilen herkömmlicher Verfahren behaftet sein.

Eine weitere Aufgabe der vorliegenden Erfindung ist es, ein Hydroxyalkyl(meth)acrylester-Produkt bereitzustellen, welches die üblichen Anforderungen zur Reinheit und zum Gehalt an Nebenkomponenten erfüllt oder welches mit möglichst geringem Aufwand weiter aufgereinigt werden kann, so dass es die entsprechenden Anforderungen erfüllt. Insbesondere soll ein Hydroxyalkyl(meth)acrylester-Produkt bereitgestellt werden, das einen möglichst geringen Gehalt an vernetzenden Nebenprodukten (Verbindungen mit zwei oder mehr C-C-Doppelbindungen), z.B. Ethylendimethacrylat, aufweist.

Es wurde überraschend gefunden, dass die oben beschriebenen Aufgaben durch das erfindungsgemäße Verfahren gelöst werden. Insbesondere wurde gefunden, dass Methacrolein und Ethylenglykol als Edukte bei der Herstellung von Hydroxyethylmethacrylat (HEMA) genutzt werden können, wobei zunächst unter Wasserabspaltung ein zyklisches Acetal gebildet wird, welches anschließend mit Sauerstoff katalytisch oxidiert wird, wodurch selektiv HEMA erhalten wird. Ethylenglykol und andere mehrwertige Alkohole sind typischerweise kostengünstig und weltweit verfügbar. Die Herstellung von Methacrolein aus Propionaldehyd und Formalin ist dem Fachmann bekannt und wird großtechnisch umgesetzt. Darüber hinaus kann Methacrolein durch Oxidation von Isobuten oder ausgehend von t-Butanol erhalten werden. Verfahren zur Herstellung von Methacrolein sind beispielsweise in Ullmanns Encyclopedia of Industrial Chemistry, 2012, Wiley-VCH Verlag GmbH, Weinheim (DOI: 10.1002/14356007.a01_149.pub2) beschrieben.

### Beschreibung der Erfindung

Die vorliegende Erfindung betrifft ein Verfahren zur Herstellung von Hydroxyalkyl-(meth)acrylestern umfassend die Schritte:
a. Umsetzung von (Meth)acrolein mit mindestens einem mehrwertigen Alkohol in Anwesenheit eines ersten Katalysators K1, wobei ein erstes Reaktionsprodukt, enthaltend mindestens ein zyklisches Acetal, erhalten wird;
b. zumindest teilweise Entfernen von Wasser aus dem ersten Reaktionsprodukt;
c. Umsetzung des ersten Reaktionsproduktes mit Sauerstoff in Gegenwart eines zweiten Katalysators K2, wobei ein zweites Reaktionsprodukt, enthaltend mindestens einen Hydroxyalkyl(meth)acrylester, erhalten wird.

In einer bevorzugten Ausführungsform handelt es sich um ein kontinuierliches oder halbkontinuierliches, bevorzugt um ein kontinuierliches, Verfahren zur Herstellung von Hydroxyalkyl(meth)acrylestern.

Der Ausdruck "(Meth)acrylat" bzw. "(Meth)acrylester" umfasst im Sinne der Erfindung Acrylat und/oder Methacrylat. Entsprechend umfasst der Ausdruck "(Meth)acrolein" im Sinne der Erfindung Acrolein und/oder Methacrolein.

In einer bevorzugten Ausführungsform wird Ethylenglykol als mehrwertiger Alkohol eingesetzt. Bevorzugt handelt es sich bei dem Hydroxyalkyl(meth)acrylester um Hydroxyethylmethacrylester (HEMA), insbesondere handelt es sich bei dem zyklischen Acetal um Methacrolein-Ethylenglykol-Acetal (2-Isopropenyl-1,3-dioxolan).

Weiterhin umfasst die Erfindung den Einsatz von alkyl-substituierten Glykolen als mehrwertiger Alkohol anstelle von Ethylglykol. Insbesondere bezieht sich die Erfindung ebenfalls auf die Herstellung von Hydroxypropyl(meth)acrylester, insbesondere 2-Hydroxypropyl(meth)acrylester (HPMA), wobei insbesondere Propandiol, z.B. 1,2-Propandiol, als mehrwertiger Alkohol eingesetzt wird.

### Schritt a - Umsetzung zum zyklischen Acetal

Das erfindungsgemäße Verfahren umfasst in Schritt a die Umsetzung von (Meth)acrolein mit mindestens einem mehrwertigen Alkohol in Gegenwart eines ersten Katalysators K1, wobei ein erstes Reaktionsprodukt, enthaltend mindestens ein zyklisches Acetal, erhalten wird. Typischerweise wird Wasser als weiteres Produkt der Acetalbildung erhalten.

Im Sinne der vorliegenden Erfindung ist ein mehrwertiger Alkohol eine Verbindung, insbesondere eine organische Verbindung, die zwei oder mehr Hydroxygruppen (-OH) umfasst. Bevorzugt handelt es sich bei dem mehrwertigen Alkohol um mindestens einen Alkohol umfassend 2 bis 10 Kohlenstoffatome, bevorzugt 2 bis 5 Kohlenstoffatome, besonders bevorzugt 2 bis 3 Kohlenstoffatome, und umfassend zwei oder mehr Hydroxygruppen, bevorzugt zwei oder drei Hydroxygruppen, besonderes bevorzugt zwei Hydroxygruppen. Bevorzugt können die Hydroxygruppen konstitutionell in 1,2-; 1,3- oder 1,4-Position im mehrwertigen Alkohol, z.B. im Diol, vorliegen. Besonders bevorzugt liegen die Hydroxygruppen in 1,2-Position im mehrwertigen Alkohol vor. Darüber hinaus kann der mehrwertige Alkohol weitere funktionelle Gruppe, beispielsweise Alkoxy-Gruppen, Aryl-Gruppen, Phosphonat-Gruppen, Phosphat-Gruppen, Alkenyl-Gruppen, Alkinyl-Gruppen, maskierte Carbonylgruppen oder Estereinheiten aufweisen.

Insbesondere bevorzugt ist der mindestens eine mehrwertige Alkohol ausgewählt aus Ethylenglykol, Propylenglykol, Butandiol und/oder Glycerin. In einer bevorzugten Ausführungsform ist der mindestens eine mehrwertige Alkohol Ethylenglykol. In einer bevorzugten Ausführungsform wird ausschließlich ein mehrwertiger Alkohol wie oben beschrieben eingesetzt.

Bevorzugt weist das zyklische Acetal eine Struktur gemäß Formel (I) auf: 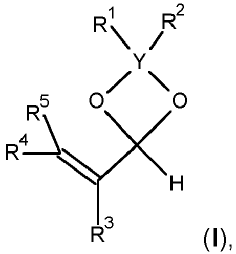 wobei Y eine C₂-C₁₀-Alkylengruppe, bevorzugt eine C₂-C₄-Alkylengruppe, besonders bevorzugt eine C₂-C₃-Alkylengruppe, ist; R¹ und R² unabhängig voneinander ausgewählt sind aus H, C₁-C₂₀-Alkyl, C₁-C₂₀-Hydroxyalkyl, C₁-C₂₀-Alkoxy, und C₆-C₂₀-Aryl; R³ H oder C₁-C₂₀-Alkyl, bevorzugt H oder Methyl, ist; R⁴ und R⁵ unabhängig voneinander ausgewählt sind aus H, C₁-C₂₀-Alkyl, C₁-C₂₀-Hydroxyalkyl und C₆-C₂₀-Aryl. Bevorzugt sind R¹ und R² unabhängig voneinander ausgewählt aus H, C₁-C₆-Alkyl, C₁-C₆-Hydroxyalkyl und C₆-C₁₂-Aryl. Bevorzugt sind R⁴ und R⁵ unabhängig voneinander ausgewählt aus H, C₁-C₆-Alkyl, C₁-C₆-Hydroxyalkyl und C₆-C₁₂-Aryl, besonders bevorzugt aus H und C₁-C₆-Alkyl. Insbesondere bevorzugt sind R⁴ und R⁵ H. Insbesondere bevorzugt ist R³ Methyl.

Insbesondere bevorzugt weist das zyklische Acetal eine Struktur gemäß Formel (II) auf: 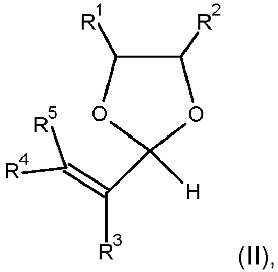 mit R¹, R², R³, R⁴ und R⁵ wie oben definiert.

Typischerweise handelt es sich bei der Reaktion von (Meth)acrolein mit mindestens einem mehrwertigen Alkohol unter Bildung des zyklischen Acetals in Schritt a um eine Gleichgewichtsreaktion, wobei insbesondere der Umsatz bei 10 bis 75 %, bevorzugt 15 bis 50 %, liegt. Typischerweise bezieht sich der Umsatz bei einem kontinuierlichen Verfahren auf den Umsatz pro Durchgang in Schritt a.

Bevorzugt erfolgt die Umsetzung in Schritt a in Gegenwart mindestens einer sauren Verbindung als Katalysator K1, insbesondere ausgewählt aus Brönsted-Säuren und Lewis-Säuren. Bevorzugt ist der mindestens eine Katalysator K1 ausgewählt aus der Gruppe bestehend aus Phosphorsäure, Schwefelsäure, Sulfonsäuren, Carbonsäuren (z.B. Ameisensäure, Essigsäure), Lanthanoid-Salzen, Metallsalzen der Elemente der Gruppen 3 bis 15 des Periodensystems, und Ionenaustauscherharzen enthaltend mindestens eine saure Gruppe ausgewählt aus Phosphonsäure (-P(=O)(OH)₂), Sulfonsäuren (-S(=O)₂OH) und Carbonsäuren (-COOH) (z.B. Essigsäure (-CH₂-CH₂-C(=O)OH)). Insbesondere bevorzugt ist der mindestens eine Katalysator K1 ausgewählt aus der Gruppe bestehend aus Phosphorsäure, Schwefelsäure, Sulfonsäuren, Carbonsäuren (z.B. Ameisensäure, Essigsäure) und Ionenaustauscherharzen enthaltend mindestens eine saure Gruppe ausgewählt aus Sulfonsäuren (-S(=O)₂OH) und Carbonsäuren (-COOH).

Es ist zudem möglich mindestens eine Lewis-Säure als Katalysator K1 einzusetzen, beispielsweise ausgewählt aus Lanthanoid-Salzen und Metallsalzen der Elemente der Gruppen 3 bis 15 des Periodensystems, insbesondere kann es sich bei den Verbindungen um Halogenide, Hydroxide, Mesilate, Triflate, Carboxylate und/oder Chalkogenide handeln. Beispielsweise kann mindestens eine Lewis-Säure als Katalysator K1 eingesetzt werden ausgewählt aus Halogeniden, Hydroxiden, Mesilaten, Triflaten, Carboxylate und Chalkogeniden (bevorzugt ausgewählt aus Halogeniden) von Alkalimetallen (insbesondere Li⁺, Na⁺, K⁺), Erdalkalimetallen (insbesondere Be²⁺, Mg²⁺, Ca²⁺), B³⁺, Al³⁺, In³⁺, Sn²⁺, Sn⁴⁺, Si⁴⁺, Sc³⁺, Ti⁴⁺, Pd²⁺, Ag⁺, Cd²⁺, Pt²⁺, Au⁺, Hg²⁺, In³⁺, Tl³⁺, Pb²⁺; und aus organische Salzen, z.B. Alkoholaten, von Ti⁴⁺, Sn⁴⁺ und B³⁺, z.B. Ti(OH)₄, B(OR)₃, Sn(OR)₄, mit R ist C₁-C₁₀-Alkyl. Beispielsweise kann als Katalysator K1 bevorzugt eine bekannte Lewis-Säure, ausgewählt aus BCl₃, BF₃, B(OH)₃, B(CH₃)₃, AlCl₃, AlF₃, TiCl₄, Ti(OH)₄, ZnCl₂, SiBr₄, SiF₄, PF₅, Ti(OR)₄, B(OR)₃, und Sn(OR)₄, mit R ist C₁-C₁₀-Alkyl, eingesetzt werden.

Bevorzugt handelt es sich bei dem Katalysator K1 um eine oder mehrere Brönsted-Säuren, welche bevorzugt einen pK_{S}-Wert von kleiner oder gleich 5, besonders bevorzugt von kleiner oder gleich 2 aufweist.

Bevorzugt umfasst der Katalysator K1 mindestens eine saure Gruppe, welche einen pKs-Wert von kleiner oder gleich 5, besonders bevorzugt von kleiner oder gleich 2 aufweist.

In einer bevorzugten Ausführungsform erfolgt die Umsetzung in Schritt a in Gegenwart mindestens einer sauren Verbindung als Katalysator K1, ausgewählt aus Brönsted-Säuren und Lewis-Säuren, wobei der Katalysator in heterogener oder homogener Form vorliegt.

Weiterhin bevorzugt handelt es sich bei dem Katalysator K1 um einen heterogenen Katalysator. Beispielsweise kann der Katalysator auf einer Polymermatrix, Kompositmatrix und/oder einem oxidischen Trägermaterial angebracht sein. Ebenso bevorzugt ist der Einsatz von heterogenen Polysäuren, zum Beispiel auf Basis von Molybdaten. Vorteilhaft an der Verwendung eines heterogenen Katalysators K1 ist insbesondere, dass sich der Katalysator K1 und das Reaktionsgemisch leichter voneinander trennen lassen, und/oder dass der Katalysator K1 über längere Zeit wieder verwendet (bzw. allgemein betrieben) werden kann.

In einer bevorzugten Ausführungsform erfolgt die Umsetzung von (Meth)acrolein mit dem mindestens einen mehrwertigen Alkohol in Schritt a bei einem molare Verhältnis von (Meth)acrolein zu mehrwertigen Alkohol(en) im Bereich von 1:50 bis 50:1, bevorzugt von 1:10 bis 10:1, besonders bevorzugt von 1:3 bis 3:1.

Bevorzugt erfolgt die Umsetzung von (Meth)acrolein mit dem mehrwertigen Alkohol in Schritt a in einem Temperaturbereich von -50 °C bis 100 °C, besonders bevorzugt von -10 °C bis 30 °C, insbesondere bevorzugt 0 bis 20 °C. Bevorzugt erfolgt die Umsetzung von (Meth)acrolein mit dem mehrwertigen Alkohol in Schritt a in einem Druckbereich von 0,5 bis 10 bar (absolut), besonders bevorzugt von 1 bis 5 bar (absolut). Insbesondere erfolgt die Umsetzung in Schritt a bei einer Temperatur und/oder bei einem Druck innerhalb der oben angegebenen Bereiche.

In einer bevorzugten Ausführungsform kann die Umsetzung von (Meth)acrolein mit dem mehrwertigen Alkohol (beispielsweise Ethylenglykol) in Schritt a in Gegenwart eines Lösungsmittels durchgeführt werden. Typischerweise kann ein Lösungsmittel ausgewählt aus linearen oder zyklischen Alkanen (z.B. Hexan, Octan, Cyclohexan), aromatischen Kohlenwasserstoffen (z.B. Toluol, Benzol), halogenierten Kohlenwasserstoffen (z.B. Chloroform, Tetrachlormethan, Hexachlorethan, Hexachlorcyclohexan, Chlorbenzol), Alkoholen (z.B. Methanol, Ethanol, n-Butanol, tert-Butanol), Ethern (z.B. Diisopropylether, Tetrahydrofuran, 1,3-Dioxan) oder Mischungen hiervon, eingesetzt werden. Dem Fachmann sind zudem weitere polare Lösungsmittel bekannt, welche sich in der Reaktion nach Schritt a chemisch inert verhalten. Typischerweise erfolgt die Umsetzung in Schritt a in einer Reaktionsmischung enthaltend 1 bis 90 Gew.-%, bevorzugt 5 bis 50 Gew.-%, bezogen auf die gesamte Reaktionsmischung, mindestens eines Lösungsmittels.

In einer besonders bevorzugten Ausführungsform wird die Umsetzung in Schritt a ohne Lösungsmittel durchgeführt.

### Schritt b - Entfernen von Wasser aus dem ersten Reaktionsprodukt

Das erfindungsgemäße Verfahren umfasst in Schritt b das zumindest teilweise Entfernen des Reaktionsproduktes Wasser aus dem ersten Reaktionsprodukt. Bevorzugt umfasst Schritt b des erfindungsgemäßen Verfahrens zudem das zumindest teilweise Entfernen des nicht umgesetzten, mehrwertigen Alkohols, z.B. Ethylenglykol, und/oder des nicht umgesetzten (Meth)acroleins aus dem ersten Reaktionsprodukt erhalten in Schritt a.

Bevorzugt erfolgt das Entfernen von Wasser und optional mehrwertigem Alkohol und optional (Meth)acrolein durch mindestens einen Destillationsschritt (insbesondere in Form einer Destillationskolonne, z.B. Kolonnen 7 und 10).

In einer bevorzugten Ausführungsform umfasst Schritt b, dass nicht umgesetztes (Meth)acrolein und/oder nicht umgesetzter mehrwertiger Alkohol, typischerweise zusammen mit dem Wasser, vom ersten Reaktionsprodukt abgetrennt werden, gegebenenfalls vom Wasser getrennt werden, und in die Umsetzung in Schritt a zurückgeführt werden.

Insbesondere bevorzugt umfasst Schritt b, dass in einem ersten Trennschritt, bevorzugt in einem Destillationsschritt, (Meth)acrolein und zumindest teilweise Wasser, vom ersten Reaktionsprodukt abgetrennt werden. Insbesondere erfolgt in diesem ersten Trennschritt eine destillative Trennung von (Meth)acrolein und dessen Azeotrop mit Wasser vom ersten Reaktionsprodukt. Insbesondere erfolgt der erste Trennschritt in einer Destillationskolonne (z.B. Kolonne 7).

Weiterhin bevorzugt umfasst Schritt b, dass in mindestens zwei Trennschritten, bevorzugt zwei Destillationsschritten, Wasser, (Meth)acrolein und mehrwertiger Alkohol vom ersten Reaktionsprodukt, enthaltend mindestens ein zyklisches Acetal, abgetrennt werden.

Insbesondere bevorzugt umfasst Schritt b, dass (z.B. in einem zweiten Trennschritt) der mehrwertige Alkohol und gegebenenfalls Wasser sowie gegebenenfalls Schwersieder, zumindest teilweise vom Reaktionsprodukt abgetrennt werden, bevorzugt in einem Destillationsschritt (z.B. Kolonne 10).

In einer bevorzugten Ausführungsform umfasst Schritt b, dass in mindestens zwei Trennschritten Wasser, nicht umgesetztes (Meth)acrolein und nicht umgesetzter mehrwertiger Alkohol vom ersten Reaktionsprodukt, enthaltend mindestens ein zyklisches Acetal, abgetrennt werden, wobei in einem ersten Trennschritt, bevorzugt in einem Destillationsschritt (z.B. Kolonne 7), (Meth)acrolein und zumindest teilweise Wasser (z.B. (Meth)acrolein und dessen Azeotrop mit Wasser), vom ersten Reaktionsprodukt abgetrennt werden, und wobei in einem zweiten Trennschritt, bevorzugt in einem Destillationsschritt (z.B. Kolonne 10), der mehrwertige Alkohol und gegebenenfalls Wasser sowie gegebenenfalls Schwersieder, zumindest teilweise vom ersten Reaktionsprodukt abgetrennt werden.

Bevorzugt erfolgt die Abtrennung des (Meth)acrolein in Schritt b in der Weise, dass in der Reaktionsmischung in Schritt c weniger als 10 Gew.-%, bevorzugt weniger als 8 Gew.-%, insbesondere bevorzugt weniger als 5 Gew.-%, (Meth)acrolein, bezogen auf die gesamte Reaktionsmischung in Schritt b, enthalten sind.

Bevorzugt erfolgt die Abtrennung von Wasser in Schritt b in der Weise, dass in der Reaktionsmischung in Schritt c weniger als 5 Gew.-%, bevorzugt weniger als 2 Gew.-%, insbesondere bevorzugt weniger als 1 Gew.-%, Wasser, bezogen auf die gesamte Reaktionsmischung in Schritt b, enthalten sind.

Bevorzugt erfolgt die Abtrennung des mehrwertigen Alkohols in Schritt b in der Weise, dass in der Reaktionsmischung in Schritt c weniger als 10 Gew.-%, bevorzugt weniger als 8 Gew.-%, insbesondere bevorzugt weniger als 5 Gew.-%, mehrwertiger Alkohol, bezogen auf die gesamte Reaktionsmischung in Schritt c, enthalten sind. Insbesondere bevorzugt erfolgt in Schritt b eine nahezu vollständige Abtrennung des mehrwertigen Alkohols vom ersten Reaktionsprodukt.

### Schritt c - Oxidation

Das erfindungsgemäße Verfahren umfasst in Schritt c die Umsetzung des ersten Reaktionsproduktes, enthaltend mindestens ein zyklisches Acetal (insbesondere 2-Isopropenyl-1,3-dioxolan), mit Sauerstoff in Gegenwart eines zweiten Katalysators K2, wobei ein zweites Reaktionsprodukt, enthaltend mindestens einen Hydroxyalkyl(meth)acrylester, erhalten wird.

Schritt c umfasst typischerweise eine oxidative Veresterung des zyklischen Acetals, das in Reaktionsstufe a erhalten wird. Typischerweise wird der Hydroxyalkyl(meth)acrylester durch oxidative Ringöffnung des zyklischen Acetals erhalten.

Bevorzugt weist der Hydroxyalkyl(meth)acrylester eine Struktur gemäß Formel (III) auf: 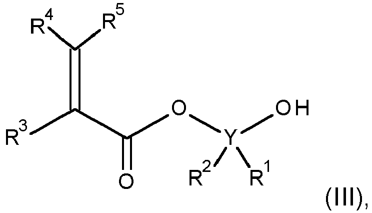 wobei Y eine C₂-C₁₀-Alkylengruppe, bevorzugt eine C₂-C₄-Alkylengruppe, besonders bevorzugt eine C₂-C₃-Alkylengruppe, ist; R¹ und R² unabhängig voneinander ausgewählt sind aus H, C₁-C₂₀-Alkyl, C₁-C₂₀-Hydroxyalkyl, C₁-C₂₀-Alkoxy und C₆-C₂₀-Aryl; R³ H oder C₁-C₂₀-Alkyl, bevorzugt H oder Methyl, ist; R⁴ und R⁵ unabhängig voneinander ausgewählt sind aus H, C₁-C₂₀-Alkyl, C₁-C₂₀-Hydroxyalkyl und C₆-C₂₀-Aryl. Bevorzugt sind R¹ und R² unabhängig voneinander ausgewählt aus H, C₁-C₆-Alkyl, C₁-C₆-Hydroxyalkyl und C₆-C₁₂-Aryl. Bevorzugt sind R⁴ und R⁵ unabhängig voneinander ausgewählt aus H, C₁-C₆-Alkyl, C₁-C₆-Hydroxyalkyl und C₆-C₁₂-Aryl, besonders bevorzugt aus H und C₁-C₆-Alkyl. Insbesondere bevorzugt sind R⁴ und R⁵ H. Insbesondere bevorzugt ist R³ Methyl.

Insbesondere bevorzugt weist der Hydroxyalkyl(meth)acrylester eine Struktur gemäß Formel (IV) auf: 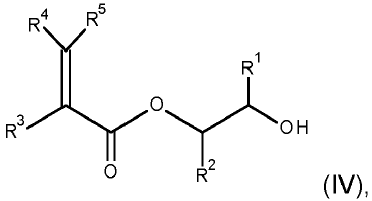 mit R¹, R², R³, R⁴, R⁵ wie oben definiert.

Bevorzugt wird die Umsetzung in Schritt c in Gegenwart eines Metall- und/oder Halbmetall-haltigen, heterogenen Katalysatorsystems als Katalysator K2, besonders bevorzugt eines Edelmetall-haltigen, heterogenen Katalysatorsystems als Katalysator K2, durchgeführt.

Im Sinne der vorliegenden Erfindung umfasst der Begriff Edelmetall die Elemente Ruthenium (Ru), Rhodium (Rh), Palladium (Pd), Osmium (Os), Iridium (Ir), Platin (Pt), Silber (Ag), Gold (Au), und Rhenium (Re), insbesondere Gold (Au), Silber (Ag) und die Platinmetalle (Ru, Rh, Pd, Os, Ir, Pt).

Bevorzugt ist der mindestens eine Katalysator K2 ein heterogener Katalysator, enthaltend ein oder mehrere Trägermaterialien und eine oder mehrere aktive Komponenten, wobei das Trägermaterial ausgewählt ist aus Aktivkohle, Siliziumdioxid, Aluminiumoxid, Titandioxid, Alkalimetalloxiden, Erdalkalimetalloxiden und Mischungen hiervon, und wobei die aktive Komponente mindestens ein Element enthält, ausgewählt aus Palladium (Pd), Platin (Pt), Iridium (Ir), Rhodium (Rh), Ruthenium (Ru), Gold (Au), Cobalt (Co), Nickel (Ni), Zink (Zn), Kupfer (Cu), Eisen (Fe), Selen (Se), Tellur (Te), Arsen (As), Antimon (Sb), Bismut (Bi), Germanium (Ge), Zinn (Sn) und Blei (Pb), wobei die Elemente elementar, als Legierung oder in Form ihrer Verbindungen in beliebiger Oxidationsstufe (bevorzugt in Form ihrer Oxide) vorliegen können.

Insbesondere handelt es sich bei dem Trägermaterial um Siliziumdioxid und/oder Aluminiumoxid, bevorzugt um Aluminiumoxid.

Besonders bevorzugt enthält die aktive Komponente mindestens ein Element, ausgewählt aus Palladium (Pd), Platin (Pt), Iridium (Ir), Rhodium (Rh), Ruthenium (Ru), Gold (Au), Bismut (Bi) und/oder Tellur (Te), wobei die Elemente elementar, als Legierung oder in Form ihrer Verbindungen in beliebiger Oxidationsstufe (bevorzugt in Form ihrer Oxide) vorliegen können. Insbesondere bevorzugt umfasst die aktive Komponente des Katalysators K2 Palladium.

Besonders bevorzugt ist der mindestens eine Katalysator K2 ein heterogenen Katalysator enthaltend Siliziumdioxid und/oder Aluminiumoxid als Trägermaterial und enthaltend mindestens ein Element, ausgewählt aus Palladium, Bismut und Tellur, als aktive Komponente, wobei die Elemente elementar, als Legierung oder in Form ihrer Verbindungen in beliebiger Oxidationsstufe (bevorzugt in Form ihrer Oxide) vorliegen können.

In einer bevorzugten Ausführungsform handelt es sich bei der aktiven Komponenten des Katalysators K2 um die Kombination von mindestens einem Edelmetall, insbesondere ausgewählt aus Gold (Au), Silber (Ag) Palladium (Pd), Platin (Pt), Iridium (Ir), Rhodium (Rh) und Ruthenium (Ru); und mindestens einem weiteren Element (Dopant), insbesondere ausgewählt aus Selen (Se), Tellur (Te), Arsen (As), Antimon (Sb), Bismut (Bi), Germanium (Ge), Zinn (Sn) und Blei (Pb), wobei die Elemente elementar, als Legierung oder in Form ihrer Verbindungen in beliebiger Oxidationsstufe (bevorzugt in Form ihrer Oxide) vorliegen können.

In einer besonders bevorzugten Ausführungsform handelt es sich bei der aktiven Komponenten des Katalysators K2 um die Kombination von Palladium (Pd) und mindestens einem weiteren Element (Dopant), ausgewählt aus Selen (Se), Tellur (Te), Antimon (Sb), und Bismut (Bi), wobei die Elemente elementar, als Legierung oder in Form ihrer Verbindungen in beliebiger Oxidationsstufe (bevorzugt in Form ihrer Oxide) vorliegen können.

Weiterhin bevorzugt ist der mindestens eine Katalysator K2 ein heterogener Katalysator, enthaltend Siliziumdioxid und/oder Aluminiumoxid als Trägermaterial und eine aktive Komponente, wobei die aktive Komponente Palladium und mindestens ein weiteres Element (Dopant), ausgewählt aus Selen (Se), Tellur (Te) und Bismut (Bi), ist, wobei die Elemente elementar, als Legierung oder in Form ihrer Oxide in beliebiger Oxidationsstufe vorliegen können.

Bevorzugt enthält der Katalysator K2 mindestens 70 Gew.-%, bevorzugt 70 bis 99,9 Gew.-%, bezogen auf die gesamte Katalysatormasse, ein oder mehrere Trägermaterialien und höchstens 30 Gew.%, bevorzugt 0,1 bis 30 Gew.-%, bezogen auf die gesamte Katalysatormasse, eine oder mehrere aktive Komponenten.

In einer bevorzugten Ausführungsform wird der heterogene zweite Katalysator K2 in Schritt c in Form einer Pulvers eingesetzt. Hierbei wird bevorzugt die Umsetzung in Schritt c in Gegenwart eines dispergierten, pulverförmigen Katalysators K2 durchgeführt.

Typischerweise beträgt die Menge an Katalysator K2 in Schritt c, bezogen auf die Gesamtmasse an Reaktionsgemisch, 0,1 bis 30 Gew.-%, bevorzugt 1,0 bis 20 Gew.-% und besonders bevorzugt 2,0 bis 15 Gew.-%.

In einer alternativen Ausführungsform wird der Schritt c in einem Festbett- oder Rieselbettreaktor durchgeführt, wobei das Verhältnis von Katalysator zu Reaktionsgemisch mit der dem Fachmann bekannten Kenngröße LHSV (Liquid-Hourly-Space-Velocity), z.B. in L Flüssigkeit / (kg Kataysator x hr) oder in kg Flüssigkeit / (kg Kataysator x hr), angegeben wird. Typischerweise beträgt die LHSV 0,05 bis 15, bevorzugt zwischen 0,1 bis 10 und besonders bevorzugt zwischen 1 und 5.

Bevorzugt wird bei der Umsetzung in Schritt c eine Reaktionsmischung, enthaltend das erste Reaktionsprodukt und den zweiten Katalysator K2, mit einem Sauerstoff-haltigen Gas in Kontakt gebracht wird. Dies kann in dem Fachmann bekannten Reaktoren oder Aggregaten in der Reaktorperipherie erreicht werden, wie z.B. Blasensäulen, begasten Rührkessel-Reaktoren und Rieselbett-Reaktoren.

Typischerweise wird bei der Umsetzung in Schritt c die Reaktionsmischung mit einem Sauerstoff-haltigen Gas in Kontakt gebracht, wobei ein Sauerstoff-haltiges Abgas erhalten wird. Typischerweise weist das Sauerstoff-haltige Abgas einen Sauerstoff-Gehalt im Bereich von 1 bis 10 Vol.-%, bevorzugt 1 bis 5 Vol.-%, bezogen auf das gesamte Sauerstoff-haltige Abgas, auf.

Typischerweise wird bei der Umsetzung in Schritt c die Reaktionsmischung mit einem Sauerstoff-haltigen Gas in Kontakt gebracht wird, wobei das Sauerstoff-haltige Gas einen Sauerstoff-Gehalt im Bereich von 1 bis 40 Vol.-%, bevorzugt 5 bis 22 Vol.-%, bezogen auf das gesamte Sauerstoff-haltige Gas, aufweist. Bevorzugt kann Luft als Sauerstoff-haltiges Gas in Schritt c eingesetzt werden.

Insbesondere bevorzugt wird bei der Umsetzung in Schritt c ein Sauerstoff-haltiges Abgas erhalten, wobei das Sauerstoff-haltige Abgas in mindestens einer Stufe abgekühlt wird, und wobei das Sauerstoff-haltige Abgas einen Sauerstoff-Gehalt im Bereich von 1 bis 10 Vol.-%, bevorzugt 1 bis 5 Vol.-%, bezogen auf das gesamte Sauerstoff-haltige Abgas, aufweist. Insbesondere kann durch eine mindestens einstufige Abkühlung eine Kondensation und Abtrennung von organischen Komponenten im Abgas erzielt werden, welche dann bevorzugt in das Verfahren zurückgeführt werden können.

In einer bevorzugten Ausführungsform wird bei der Umsetzung in Schritt c eine Reaktionsmischung, enthaltend das erste Reaktionsprodukt und den zweiten Katalysator K2, eingesetzt, die einen Gehalt an mehrwertigen Alkohol(en) von kleiner als 10 Gew.-%, bevorzugt kleiner als 8 Gew.-%, insbesondere bevorzugt kleiner als 5 Gew.-%, bezogen auf die Reaktionsmischung, aufweist.

In einer bevorzugten Ausführungsform wird bei der Umsetzung in Schritt c eine Reaktionsmischung, enthaltend das erste Reaktionsprodukt und den zweiten Katalysator K2, eingesetzt, die einen Gehalt an (Meth)acrolein von kleiner als 10 Gew.-%, bevorzugt kleiner als 8 Gew.-%, insbesondere bevorzugt kleiner als 5 Gew.-%, bezogen auf die Reaktionsmischung, aufweist.

Bevorzugt erfolgt die Umsetzung des ersten Reaktionsproduktes mit Sauerstoff in Schritt c in einem Temperaturbereich von 0 °C bis 120 °C, besonders bevorzugt von 50 °C bis 120 °C, insbesondere bevorzugt 60 °C bis 100 °C. Bevorzugt erfolgt die Umsetzung des ersten Reaktionsproduktes mit Sauerstoff in Schritt c in einem Druckbereich von 0,5 bis 50 bar (absolut), besonders bevorzugt von 1 bis 50 bar (absolut), insbesondere bevorzugt von 2 bis 30 bar (absolut). Insbesondere erfolgt die Umsetzung in Schritt c bei einer Temperatur und/oder einem Druck innerhalb der oben angegebenen Bereiche.

In einer bevorzugten Ausführungsform kann die Umsetzung des ersten Reaktionsproduktes, enthaltend mindestens ein zyklisches Acetal, mit Sauerstoff in Schritt c in Anwesenheit von einem Lösungsmittel durchgeführt werden. Typischerweise kann ein Lösungsmittel ausgewählt aus linearen oder zyklischen Alkanen (z.B. Hexan, Octan, Cyclohexan), aromatischen Kohlenwasserstoffen (z.B. Toluol, Benzol), halogenierten Kohlenwasserstoffen (z.B. Chloroform, Tetrachlormethan, Hexachlorethan, Hexachlorcyclohexan, Chlorbenzol), Alkoholen (z.B. Methanol, Ethanol, n-Butanol, tert-Butanol), Ethern (z.B. Diisopropylether, Tetrahydrofuran, 1,3-Dioxan), Estern (z.B. Essigsäuremethylester, Essigsäureethylester), Nitrilen (z.B. Acetonitril) oder Mischungen hiervon, eingesetzt werden. Bevorzugte Ester sind C₁-C₂₀-Alkylester, bevorzugt C₁-C₁₀-Alkylester, von aliphatischen und aromatischen C₁-C₂₀-Carbonsäuren, z.B. von Ameisensäure, Essigsäure, Propansäure, Butansäure, Pentansäure, Hexansäure und Benzoesäure. Typischerweise erfolgt die Umsetzung in Schritt c in einer Reaktionsmischung enthaltend 1 bis 90 Gew.-%, bevorzugt 5 bis 50 Gew.-%, bezogen auf die gesamte Reaktionsmischung, mindestens eines Lösungsmittels.

Typischerweise erfolgt die oxidative Umsetzung des zyklischen Acetals in Schritt c nicht vollständig. Bevorzugt umfasst das erfindungsgemäße Verfahren einen Aufarbeitungsschritt d, wobei das zyklische Acetal zumindest anteilig vom zweiten Reaktionsprodukt, enthaltend Hydroxyalkyl(meth)acrylester, abgetrennt wird und optional in die Umsetzung in Schritt c zurückgeführt wird. Beispielsweise kann die Abtrennung des zyklischen Acetals von dem zweiten Reaktionsprodukt in einem oder mehreren Destillationsschritten (Destillationskolonnen) erfolgen.

Das erfindungsgemäße Verfahren kann optional weitere Schritte zur Aufbereitung und/oder Reinigung des zweiten Reaktionsprodukt, enthaltend mindestens einen Hydroxyalkyl(meth)acrylester, umfassen. Typischerweise umfassen die weiteren Schritte zur Aufbereitung und/oder Reinigung Destillationsschritte und/oder Extraktionsschritte und/oder Kristallisationsschritte.

In einer bevorzugten Ausführungsform wird ein Rohprodukt enthaltend Hydroxyalkyl(meth)acrylester aus dem zweiten Reaktionsprodukt in mindestens einem Destillationsschritt gewonnen (z.B. Kolonne 16). Typischerweise können in diesem Destillationsschritt nicht umgesetztes Acetal und optional Lösungsmittel vom zweiten Reaktionsprodukt abgetrennt und optional in die Oxidation nach Schritt c) zurückgeführt werden.

In einer bevorzugten Ausführungsform erfolgen die Umsetzung in Schritt a in einem Temperaturbereich von -50 °C bis 100 °C, bevorzugt -10 °C bis 30 °C, insbesondere bevorzugt 0 °C bis 20 °C, und die Umsetzung in Schritt c in einem Temperaturbereich von 0 °C bis 120 °C, bevorzugt 50 °C bis 120 °C, insbesondere bevorzugt 60 °C bis 100 °C, wobei die Umsetzung in Schritt a bei einer Temperatur erfolgt, die um mindestens 15 °C niedriger ist als die Temperatur der Umsetzung in Schritt c.

In einer bevorzugten Ausführungsform erfolgen die Umsetzung in Schritt a in einem Druckbereich von 0,5 bis 10 bar (absolut), bevorzugt 1 bis 5 bar (absolut), und die Umsetzung in Schritt c in einem Druckbereich von 1 bis 50 bar (absolut), bevorzugt 2 bis 30 bar (absolut), wobei die Umsetzung in Schritt c bei einem Druck erfolgt, der um mindestens 0,1 bar absolut, bevorzugt um mindestens 0,5 bar absolut, höher ist als der Druck der Umsetzung in Schritt a.

Typischerweise kann das erfindungsgemäße Verfahren die Zugabe von einem oder mehreren Stabilisatoren umfassen, beispielsweise ausgewählt aus Polymerisationsinhibitoren, Radikalfängern und Antioxidantien, insbesondere Stabilisatoren wie in EP-B 1 125 919 beschrieben. Beispielsweise kann der Stabilisator ausgewählt sein aus Phenol, substituierten Phenolen (z.B. 4-Methoxyphenol), Hydrochinon, Alkyl-substituierten Hydrochinonen (z.B. Methyl-Hydrochinon, tert-Butyl Hydrochinon, 2,6-di-tert-Butyl-Parahydrochinon, 2,5-di-tert-Butyl-Hydrochinon); gesättigten Hydroxyalkyl-Carboxylaten (z.B. HydroxyethylAcetat, Hydroxyethyl-Propionat, Hydroxyethyl-Isobutyrat, Hydroxypropyl-Acetat) und N-Oxyl-Verbindungen (z.B. Piperidin-oxyl-Verbindungen, wie 4-Hydroxy-2,2,6,6-tetramethylpiperidine-1-oxyl).

Typischerweise kann die Zugabe eines Stabilisators wie oben beschrieben in einem oder mehreren der Schritte a, b und/oder c erfolgen. Ebenso kann die Zugabe eines Stabilisators wie oben beschrieben vor oder nach einem der Schritte a, b und/oder c erfolgen. Typischerweise können dem ersten Reaktionsprodukt ein oder mehrere Stabilisatoren zugesetzt werden. Die Stabilisator-Zugabe kann beispielsweise nach der Umsetzung in Schritt a, beispielsweise in Schritt b, erfolgen.

### Beschreibung der Figuren

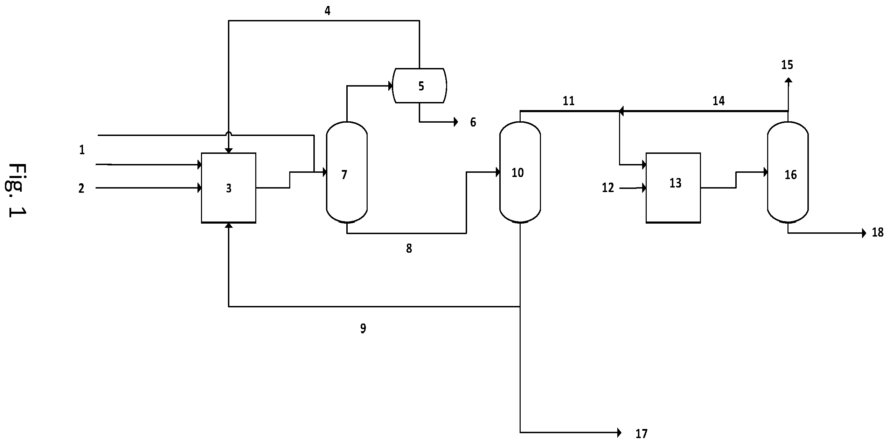

Figur 1 zeigt beispielhaft ein mögliches schematisches Fließbild des erfindungsgemäßen Verfahrens. Hierbei haben die Bezeichnungen die folgende Bedeutung:
- 1: (Meth)acrolein-Strom auf 3 oder 7
- 2: Dialkohol-Strom
- 3: Reaktor, erste Stufe, Bildung des zyklischen Acetals
- 4: optionaler Recyclingstrom ((Meth)acrolein)
- 5: Dekanter
- 6: Abtrennung Wasser
- 7: Destillationskolonne, Abtrennung (Meth)acrolein und Wasser
- 8: Roh-Strom erstes Reaktionsprodukt (zyklisches Acetal)
- 9: optionaler Recyclingstrom (Dialkohol)
- 10: Destillationskolonne, Abtrennung Acetal von Dialkohol und Schwersieder
- 11: Acetal-Strom zur zweiten Reaktionsstufe, hier optional Zugabe von Stabilisator und Lösungsmittel zur zweiten Reaktionsstufe möglich
- 12: Luftdosierung für die Oxidation des Acetals
- 13: Reaktor, zweite Stufe, Bildung des Hydroxyalkyl(meth)acrylesters
- 14: optionaler Recyclingstrom (Acetal und/oder Lösungsmittel)
- 15: Abgasleitung
- 16: Destillationskolonne, Abtrennung Acetal und/oder Lösungsmittel
- 17: Ausschleusung von Schwersiedern der ersten Reaktionsstufe
- 18: Roh-Strom zweites Reaktionsprodukt (Hydroxyalkyl(meth)acrylester)

### Experimenteller Teil

### Beispiel 1a - Kontinuierliche Herstellung des zyklischen Acetals (2-Isopropenyl-1,3-dioxolan) auf Basis von Methacrolein und Ethylenglykol / Methacrolein auf Destillationskolonne

Eingesetzt wurde ein Doppelmantelschlaufenreaktor mit einer aktiven Katalysatormenge von 5 kg und einem Gesamtvolumen von 25 L. Als Katalysator (K1) wurde ein Sulfonsäureharz von Lanxess (K2431) verwendet. Der Reaktor wurde über den Mantel (Betrieb mit ARAL Antifreeze Kühlmittel) so geregelt, dass die Innentemperatur 2-3 °C betrug. Der Reaktor war an eine Destillationskolonne angeschlossen (DN 150mm, Höhe 6m), die mit einer Packung des Typs Sulzer DX (HETP 60 mm, ∼ 16,6 theoretische Böden pro Meter Packungshöhe) bestück war.

Ethylenglykol (EG) (15 kg/h, 242 mol/h) wurde mit 100 ppm TEMPOL (4-Hydroxy-2,2,6,6-tetramethylpiperidine-1-oxyl) versetzt und direkt in den Reaktor (3) dosiert, wohingegen das Methacrolein (MAL) zusammen mit dem Reaktoraustrag auf eine Destillationskolonne (7) gefahren wurde. Dadurch konnte sowohl das Methacrolein (MAL), als auch das Reaktionsprodukt entwässert werden, was vorteilhaft für die Reaktionsführung war. Im Kopf der Kolonne sammelte sich dabei das Hetero-Azeotrop von Methacrolein und Wasser, welches nach Abkühlen in zwei Phasen zerfällt. Das Methacrolein wurde über einen Dekanter (5) zurück in den Reaktor (3) gefahren. Die Menge an frischem Methacrolein wurde so justiert, dass die Menge an Methacrolein, die in den Reaktor gefahren wurde stündlich 17,25 kg (246 mol) betrug. Das molare Verhältnis von Methacrolein zu Ethylenglykol betrug somit 1,02 und die LHSV (liquid hourly space velocity) lag bei 6,4 ((kg MAL + kg EG) / (kg Kat. * hr)).

Der Inhalt des Schlaufenreaktors wurde über eine Pumpe zirkuliert, so dass die interne Umwälzung eine gute Durchmischung der Reaktanden gewährleistete; bei niedrigen Umlaufströmen konnte die Ausbildung von zwei Phasen beobachtet werden. Zur Verbesserung der Durchmischung wurde vor dem Katalysatorbett ein statischer Mischer installiert. Die Verweilzeit im Reaktor betrug knapp 45 Minuten und das Reaktionsgemisch am Reaktor-Ausgang hatte eine Zusammensetzung von 40 Gew.-% Methacrolein, 34 Gew.-% Ethylenglykol, 21 Gew.-% Acetal, 3 Gew.-% Wasser und 2 Gew.-% Nebenkomponenten. Die Nebenkomponenten sind insbesondere hochsiedende Additionsprodukte von Glykol oder Wasser an das Acetal.

Der Reaktor wurde über einen Zeitraum von 3 Stunden angefahren und danach für 12 Stunden bei diesen Parametern kontinuierlich, stabil betrieben. Der Umsatz an Methacrolein betrug 26% und die Selektivität zum Acetal betrug 92%.

### Beispiel 1b - Kontinuierliche Abtrennung von Methacrolein und Wasser aus dem Produktgemisch aus Beispiel 1a

Der Reaktionsaustrag aus Beispiel 1a (32,25 kg/h) wurde mit dem frischen Methacrolein gemischt und in eine Destillationskolonne (7) gefahren (DN 150mm, Höhe 6m), die mit einer Packung des Sulzer DX (HETP 60 mm, ∼ 16,6 theoretische Böden pro Meter Packungshöhe) bestück war. Die Kolonne wurde bei einem Druck von 90 mbar, einer Sumpftemperatur von 90 °C, einer Destillat-Temperatur von 5 °C und einem Rückflussverhältnis von 1 betrieben. An den Kolonnenkopf war ein Dekanter (5) angeschlossen, mittels dem das erhaltene Hetero-Azeotrop von Methacrolein und Wasser (98,8 Gew.-% MAL und 1,2 Gew.-% Wasser) getrennt wurde.

Die wässrige Phase des Dekanters bestand zu 93,9 Gew.-% aus Wasser und 6,1 Gew.-% Methacrolein. Die wässrige Phase wurde diskontinuierlich angestrippt, wobei ein Methacrolein freier, wässriger Sumpf erhalten wurde. Dieser Sumpf kann biologisch behandelt oder verbrannt werden. Die organische Phase des Dekanters (5) wurde in die Reaktion (Reaktor 3) rezykliert.

Der Sumpf der Destillationskolonne (7) (18,4 kg/h) bestand aus dem zyklischen Acetal (37 Gew.-%), Ethylenglykol (60 Gew.-%) und den unter 1a genannten hochsiedenden Nebenprodukten (3 Gew.-%).

Durch die Fahrweise, in der frisches Methacrolein auf die Destillationskolonne (7) gegeben wurde, war sichergestellt, dass das Azeotrop von Methacrolein und Wasser nahezu vollständig aus dem Sumpf der Kolonne 7 entfernt wurde. Somit wurde die Abreicherung des Methacrolein im Sumpfaustrag (aus 7) auf ein Niveau unter 1000 ppm erzielt.

### Beispiel 1c - Kontinuierliche Herstellung des zyklischen Acetals (2-Isopropenyl-1,3-dioxolan) auf Basis von Methacrolein und Ethylenglykol / Methacrolein in den Reaktor

Eingesetzt wurde ein Doppelmantelschlaufenreaktor mit einer aktiven Katalysatormenge von 5 kg und einem Gesamtvolumen von 25L. Als Katalysator (K1) wurde ein Sulfonsäureharz von Lanxess (K2431) verwendet. Der Reaktor wurde über den Mantel (Betrieb mit ARAL Antifreeze Kühlmittel) so geregelt, dass die Innentemperatur 2-3 °C betrug.

Das Ethylenglykol (15 kg/h, 242 mol/h) und das Methacrolein (17,25 kg/h, 246 mol/h) wurden mit 100 ppm TEMPOL in den Reaktor (3) dosiert. Das molare Verhältnis von Methacrolein zu Ethylenglykol betrug somit 1.02 und die LHSV lag bei 6.4 ((kg MAL + kg EG) / (kg Kat. * hr)). Der Inhalt des Schlaufenreaktors wurde über eine Pumpe zirkuliert, sodass die interne Umwälzung eine gute Durchmischung der Reaktanden gewährleistete; bei niedrigen Umlaufströmen konnte die Ausbildung von zwei Phasen beobachtet werden. Zur Verbesserung der Durchmischung wurde vor dem Katalysatorbett ein statischer Mischer installiert.

Die Verweilzeit im Reaktor (3) war knapp 45 Minuten und das Reaktionsgemisch am Ausgang hatte eine Zusammensetzung von 40 Gew.-% Methacrolein, 34 Gew.-% Ethylenglykol, 21 Gew.-% Acetal, 3 Gew.-% Wasser und 2 Gew.-% Nebenkomponenten. Die Nebenkomponenten sind insbesondere hochsiedende Additionsprodukte von Glykol oder Wasser an das Acetal.

Der Reaktor wurde über einen Zeitraum von 3 Stunden angefahren und danach für 12 Stunden bei diesen Parametern kontinuierlich, stabil betrieben. Der Umsatz an Methacrolein betrug somit 26% und die Selektivität zum Acetal waren 92%.

### Beispiel 1d - Kontinuierliche Abtrennung von Methacrolein und Wasser aus dem Produktgemisch aus Beispiel 1c

Der Reaktionsaustrag aus Beispiel 1c (32,25kg/h) wurde in eine Destillationskolonne (7) gefahren (DN 150mm, Höhe 6m), die mit einer Packung des Sulzer DX (HETP 60 mm, ∼ 16,6 theoretische Böden pro Meter Packungshöhe) bestück war. Die Kolonne wurde bei einem Druck von 85 mbar, einer Sumpftemperatur von 88 °C, einer Destillattemperatur von 5 °C und einem Rückflussverhältnis von 2,5 betrieben. An den Kolonnenkopf war ein Dekanter (5) angeschlossen, mittels dem das erhaltene Hetero-Azeotrop von Methacrolein und Wasser (98,8 Gew.-% MAL und 1,2 Gew.-% Wasser) getrennt wurde.

Die wässrige Phase des Dekanters besteht zu 93,5 Gew.-% aus Wasser; 6,1 Gew.-% Methacrolein und 0,4 Gew.-% Acetal. Die wässrige Phase wurde diskontinuierlich angestrippt wobei ein Methacrolein freier, wässriger Sumpf erhalten wurde. Dieser Sumpf kann biologisch behandelt oder verbrannt werden. Die organische Phase des Dekanters wurde in die Reaktion (3) rezykliert und enthielt dabei 2,3 Gew.-% Acetal.

Der Sumpf der Destillationskolonne (7) (18,4 kg/h) bestand aus dem zyklischen Acetal (36 Gew.-%), Ethylenglykol (61 Gew.-%) und den unter 1a genannten hochsiedenden Nebenprodukten (3 Gew.-%). Zwar wurde eine Abreicherung auf unter 1000 ppm von Methacrolein und Wasser im Sumpf erreicht, aber ein Teil des Acetals ging im Dekanter (5) verloren.

### Beispiel 1e - Diskontinuierliche Herstellung des zyklischen Acetals (2-Isopropenyl-1,3-dioxolan) auf Basis von Methacrolein und Ethylenglykol / Zuhilfenahme eines inerten, azeotropen Schleppmittels

In einer Glasapparatur mit aufgesetzter Dean-Stark-Apparatur wurden 315 g Methacrolein (4,5 mol), 279 g Ethylenglykol (4,5 mol), 500 g Hexan und 5,2 g Phosphorsäure (1 mol%) vorgelegt. Die Reaktionsmischung wurde mit jeweils 0,4 g TEMPOL und 0,4 g 4-Methoxyphenol stabilisiert. Das Gemisch wurde für 6 Stunden auf 80 °C erhitzt, so dass in der Reaktion freiwerdendes Wasser durch Hexan als Schleppmittel entzogen wurde. Im Abtrennungsteil der Dean-Stark-Apparatur sammelte sich zudem eine wasserreiche Fraktion an und die kondensierte organische Fraktion bestehend aus Hexan und Methacrolein wurde permanent in den Reaktionsteil zurückgeführt. Nach 6 Stunden wurde die Reaktion abgebrochen.

Der Umsatz an Methacrolein lag bei ca. 70% und die Selektivität lag bei ca. 48%. Schon kurz nach Beginn der Reaktion bildete sich im Reaktionsgefäß an der Phasengrenze von Methacrolein/Hexan und Ethylenglykol eine dunkle gefärbte Trübung aus, die mit fortschreitender Reaktion zunahm. Die Trübung wurde dabei insbesondere durch hochsiedende Polymere aus Methacrolein und Glykol bzw. Additionsprodukte von Glykol an die 4-Position von Methacrolein oder dessen Acetal verursacht. Bei Erhöhung der Katalysatormenge oder Skalierung der Reaktion in einen größeren Maßstab beschleunigte sich die Bildung dieser Hochsieder.

Grundsätzlich wurden in dieser Verfahrensweise unbefriedigende Ausbeuten an Acetal erhalten, vor allem in Hinsicht auf die Raum-Zeit-Ausbeute. Das Prinzip der Wasserabtrennung mittels Schleppmittel und mittels Methacrolein-Wasser-Azeotrop Abtrennung ist jedoch funktional.

### Beispiel 2 - Synthese des Katalysators (K2) für die Oxidation von 2-Isopropenyl-1,3-dioxolan zu Hydroxyethylmethacrylat (HEMA)

### Beispiel 2a

In einer Glasapparatur wurden 0,90 g Bismutnitrat-Pentahydrat und 0,36 g Tellursäure unter Rühren suspendiert. Es wurde tropfenweise HNO₃ (60%) zugegeben bis alles gelöst war und die Bildung von instabilen Suboxiden unterbunden war. Es wurden 20,0 g Palladium auf Alumina (5 Gew.-% Pd) hinzugefügt und die Suspension auf 60 °C erhitzt. Nach dem Erreichen der Temperatur wurde für eine Stunde gerührt. Tropfenweise wurden 10,0 g einer Hydrazin Monohydrat Lösung hinzugeführt. Die Suspension wurde auf 90 °C erhitzt und für eine weitere Stunde gerührt. Nach Abkühlen auf Raumtemperatur wurde der schwarze Feststoff abfiltriert und mit 4 Portionen zu je 100 mL destilliertem Wasser gewaschen. Die Leitfähigkeit der letzten Waschlösung lag bei unter 100 µS/cm, was zeigte, dass die Dopanten quasi quantitativ aufgenommen wurden.

Der Feststoff wurde für 10 h bei 105 °C getrocknet und der finale Katalysator wurde erhalten. Die Stöchiometrie war Pd1,00Bi0,20Te0,17@Al2O3.

### Beispiel 2b

Die Synthese erfolgte analog zu Beispiel 2a ohne Zugabe von Tellursäure.

### Beispiel 2c

Die Synthese erfolgte analog zu Beispiel 2a ohne Zugabe von Bismutnitrat-Pentahydrat.

### Beispiel 2d

Die Synthese erfolgte analog zu Beispiel 2a mit Zugabe der doppelten Menge an Tellursäure.

### Beispiel 2e

Die Synthese erfolgte analog zu Beispiel 2a mit Zugabe der doppelten Menge an Bismutnitrat-Pentahydrat.

### Beispiel 2f

Die Synthese erfolgte analog zu Beispiel 2a mit Zugabe der doppelten Mengen an Bismutnitrat-Pentahydrat und Tellursäure.

### Beispiel 3 - Oxidation von 2-Isopropenyl-1,3-dioxolan zu Hydroxyethylmethacrylat (HEMA)

### Beispiel 3a

In einen 130 mL Stahlautoklaven mit Rührorgan wurden 400 mg Katalysator (K2) aus Beispiel 2a sowie eine 25 gew.-% Lösung von 2-Isopropenyl-1,3-dioxolan in Toluol, stabilisiert mit 200 ppm TEMPOL, eingewogen. Der Autoklav wurde verschlossen, mit 7% Sauerstoff in Stickstoff auf 37 bar aufgepresst (0,6 Äquivalente Sauerstoff pro Äquivalent Acetal) und in ein vortemperiertes Ölbad bei 70 °C gestellt. Die Reaktion wurde für 4 h gerührt und dann durch Kühlung mittels Trockeneis abgebrochen. Der Autoklav wurde vorsichtig entspannt und das Reaktionsgemisch per Gaschromatographie (GC) analysiert.

Der Umsatz lag bei 54 % und die Selektivität zu 2-Hydroxyethylmethacrylat lag bei 84%. Dies entspricht einer Raum-Zeit-Ausbeute von 3,8 mol HEMA / kg Katalysator pro Stunde. Ethylendimethacrylat konnte mittels Gaschromatographie (GC) nicht detektiert werden.

### Beispiel 3b

Die Oxidation wurde wie in Beispiel 3a durchgeführt, jedoch wurde als Lösungsmittel Ethylacetat eingesetzt. Nach 2 Stunden Reaktionszeit lag der Umsatz bei 91%, die Selektivität bei 88% und die Raum-Zeit-Ausbeute bei 19,6 mol HEMA / kg Katalysator pro Stunde. Ethylendimethacrylat konnte mittels Gaschromatographie nicht detektiert werden.

### Beispiel 3c

Die Oxidation wurde wie in Beispiel 3a durchgeführt, jedoch mit dem Katalysator aus Beispiel 2b. Der Umsatz lag bei 12%, die Selektivität bei 79%.

### Beispiel 3d

Die Oxidation wurde wie in Beispiel 3a durchgeführt, jedoch mit dem Katalysator aus Beispiel 2c Der Umsatz lag bei 73%, die Selektivität bei 78% und die Raum-Zeit-Ausbeute bei 7,1 mol HEMA / kg Katalysator pro Stunde.

### Beispiel 3e

Die Oxidation wurde wie in Beispiel 3a durchgeführt, jedoch mit dem Katalysator aus Beispiel 2d. Der Umsatz lag bei 20%, die Selektivität bei 84% und die Raum-Zeit-Ausbeute bei 2,1 mol HEMA / kg Katalysator pro Stunde.

### Beispiel 3f

Die Oxidation wurde wie in Beispiel 3a durchgeführt, jedoch mit dem Katalysator aus Beispiel 2e. Der Umsatz lag bei 83%, die Selektivität bei 59% und die Raum-Zeit-Ausbeute bei 6,1 mol HEMA / kg Katalysator pro Stunde.

### Beispiel 3g

Die Oxidation wurde wie in Beispiel 3a durchgeführt, jedoch mit dem Katalysator aus Beispiel 2f. Der Umsatz lag bei 59%, die Selektivität bei 71% und die Raum-Zeit-Ausbeute bei 5,3 mol HEMA / kg Katalysator pro Stunde.

### Beispiel 3h

Die Oxidation wurde wie in Beispiel 3a durchgeführt, jedoch wurde als Lösungsmittel Ethylenglykol eingesetzt. Nach 2 Stunden Reaktionszeit lag der Umsatz bei 99%, die Selektivität bei 55% und die Raum-Zeit-Ausbeute bei 7,06 mol HEMA / kg Katalysator pro Stunde.

Als Hauptnebenreaktion wurde die Hydrierung von 2-Hydroxyethylmethacrylat beobachtet. Die Selektivität dieser Reaktion betrug 30%. Das Beispiel demonstriert, dass für die Reaktionsführung und zur Erzielung hoher Selektivitäten und Ausbeuten vorteilhaft ist, die Konzentration an Alkohol (Edukt erste Stufe) zu kontrollieren und zu reduziert, da sonst die Nebenreaktion zum ungewünschten, hydrierten Nebenprodukt zunehmen kann.

### Beispiel 3i

Die Oxidation wurde wie in Beispiel 3a durchgeführt, jedoch wurde die Reaktion bei Atmosphärendruck durchgeführt und die Gasmenge so gewählt, dass weiterhinein Überschuss an Sauerstoff vorhanden war. Nach 4 Stunden Reaktionszeit war nahezu kein Umsatz vorhanden. Die Reaktionszeit wurde auf 48 Stunden verlängert, wobei ein Umsatz von ca. 50% gefunden wurde, die Selektivität lag bei 83%.

Somit zeigt sich, dass die Reaktion bei niedrigeren Drücken zwar möglich, aber wirtschaftlich unrentabel ist.

### Beispiel 3j

Die Oxidation wurde wie in Beispiel 3a durchgeführt, jedoch wurde die Reaktion bei 50 °C durchgeführt. Nach 4 Stunden Reaktionszeit war Umsatz 29% und die Selektivität war 83%. Bei Senkung der Temperatur wurde eine nahezu lineare Abnahme der Reaktionsgeschwindigkeit gefunden.

Somit zeigt sich, dass die Reaktion bei niedrigen Temperaturen zwar möglich, aber wirtschaftlich unrentabel ist.

## Patentansprüche

1. Verfahren zur Herstellung von Hydroxyalkyl(meth)acrylestern umfassend die Schritte:
a. Umsetzung von (Meth)acrolein mit mindestens einem mehrwertigen Alkohol in Anwesenheit eines ersten Katalysators K1, wobei ein erstes Reaktionsprodukt, enthaltend mindestens ein zyklisches Acetal, erhalten wird;
b. zumindest teilweise Entfernen von Wasser aus dem ersten Reaktionsprodukt;
c. Umsetzung des ersten Reaktionsproduktes mit Sauerstoff in Gegenwart eines zweiten Katalysators K2, wobei ein zweites Reaktionsprodukt, enthaltend mindestens einen Hydroxyalkyl(meth)acrylester, erhalten wird.

2. Verfahren gemäß Anspruch 1, **dadurch gekennzeichnet, dass** es sich um ein kontinuierliches Verfahren zur Herstellung von Hydroxyalkyl(meth)acrylestern handelt.

3. Verfahren gemäß Anspruch 1 oder 2, **dadurch gekennzeichnet, dass** die Umsetzung in Schritt a in Gegenwart mindestens einer sauren Verbindung als Katalysator K1, ausgewählt aus Brönsted-Säuren und Lewis-Säuren, erfolgt, wobei der Katalysator K1 in heterogener oder homogener Form vorliegt und mindestens eine saure Gruppen umfasst, welche einen pKs-Wert von kleiner oder gleich 5 aufweist.

4. Verfahren gemäß einem der Ansprüche 1 bis 3, **dadurch gekennzeichnet, dass** der mindestens eine Katalysator K1 ausgewählt ist aus der Gruppe bestehend aus Phosphorsäure, Schwefelsäure, Sulfonsäuren, Carbonsäuren und Ionenaustauscherharzen enthaltend mindestens eine saure Gruppe ausgewählt aus Sulfonsäuren und Carbonsäuren.

5. Verfahren gemäß einem der Ansprüche 1 bis 4, **dadurch gekennzeichnet, dass** die Umsetzung in Schritt a bei einem molare Verhältnis von (Meth)acrolein zu mehrwertigen Alkohol(en) im Bereich von 1:50 bis 50:1, bevorzugt von 1:10 bis 10:1, besonders bevorzugt von 1:3 bis 3:1, erfolgt.

6. Verfahren gemäß einem der Ansprüche 1 bis 5, **dadurch gekennzeichnet, dass** in Schritt b nicht umgesetztes (Meth)acrolein und/oder nicht umgesetzter mehrwertiger Alkohol vom ersten Reaktionsprodukt abgetrennt und in die Umsetzung nach Schritt a zurückgeführt werden.

7. Verfahren gemäß einem der Ansprüche 1 bis 6, **dadurch gekennzeichnet, dass** in Schritt b in einem ersten Trennschritt (Meth)acrolein und zumindest teilweise Wasser, vom ersten Reaktionsprodukt abgetrennt werden.

8. Verfahren gemäß einem der Ansprüche 1 bis 7, **dadurch gekennzeichnet, dass** in Schritt b in mindestens zwei Trennschritten Wasser, nicht umgesetztes (Meth)acrolein und nicht umgesetzter mehrwertiger Alkohol vom ersten Reaktionsprodukt, enthaltend mindestens ein zyklisches Acetal, abgetrennt werden.

9. Verfahren gemäß einem der Ansprüche 1 bis 8, **dadurch gekennzeichnet, dass** die Umsetzung in Schritt c in Gegenwart eines Metall- und/oder Halbmetall-haltigen, heterogenen Katalysatorsystems als Katalysator K2 durchgeführt wird.

10. Verfahren gemäß einem der Ansprüche 1 bis 9, **dadurch gekennzeichnet, dass** der mindestens eine Katalysator K2 ein heterogener Katalysator ist, enthaltend ein oder mehrere Trägermaterialien und eine oder mehrere aktive Komponenten, wobei das Trägermaterial ausgewählt ist aus Aktivkohle, Siliziumdioxid, Aluminiumoxid, Titandioxid, Alkalimetalloxiden, Erdalkalimetalloxiden und Mischungen hiervon, und wobei die aktive Komponente mindestens ein Element enthält, ausgewählt aus Palladium, Platin, Iridium, Rhodium, Ruthenium, Gold, Cobalt, Nickel, Zink, Kupfer, Eisen, Selen, Tellur, Arsen, Antimon, Bismut, Germanium, Zinn und Blei, wobei die Elemente elementar, als Legierung oder in Form ihrer Verbindungen in beliebiger Oxidationsstufe vorliegen können.

11. Verfahren gemäß einem der Ansprüche 1 bis 10, **dadurch gekennzeichnet, dass** der mindestens eine Katalysator K2 ein heterogenen Katalysator ist, enthaltend Siliziumdioxid und/oder Aluminiumoxid als Trägermaterial und enthaltend mindestens ein Element, ausgewählt aus Palladium, Bismut und Tellur, als aktive Komponente, wobei die Elemente elementar, als Legierung oder in Form ihrer Verbindungen in beliebiger Oxidationsstufe vorliegen können.

12. Verfahren gemäß einem der Ansprüche 1 bis 11, **dadurch gekennzeichnet, dass** der mindestens eine Katalysator K2 ein heterogenen Katalysator ist, enthaltend Siliziumdioxid und/oder Aluminiumoxid als Trägermaterial und eine aktive Komponente, wobei die aktive Komponente Palladium und mindestens ein weiteres Element, ausgewählt aus Selen, Tellur und Bismut, ist, wobei die Elemente elementar, als Legierung oder in Form ihrer Oxide in beliebiger Oxidationsstufe vorliegen können.

13. Verfahren gemäß einem der Ansprüche 1 bis 12, **dadurch gekennzeichnet, dass** bei der Umsetzung in Schritt c eine Reaktionsmischung, enthaltend das erste Reaktionsprodukt und den zweiten Katalysator K2, mit einem Sauerstoff-haltigen Gas in Kontakt gebracht wird, wobei in Schritt c ein Sauerstoff-haltiges Abgas erhalten wird, wobei das Sauerstoff-haltige Abgas in mindestens einer Stufe abgekühlt wird, und wobei das Sauerstoff-haltige Abgas einen Sauerstoff-Gehalt im Bereich von 1 bis 10 Vol.-%, bezogen auf das gesamte Sauerstoff-haltige Abgas, aufweist.

14. Verfahren gemäß einem der Ansprüche 1 bis 13, **dadurch gekennzeichnet, dass** bei der Umsetzung in Schritt c eine Reaktionsmischung, enthaltend das erste Reaktionsprodukt und den zweiten Katalysator K2, eingesetzt wird, die einen Gehalt an mehrwertigen Alkohol(en) von kleiner 10 Gew.-%, bezogen auf die Reaktionsmischung in Schritt c, aufweist.

15. Verfahren gemäß einem der Ansprüche 1 bis 14, **dadurch gekennzeichnet, dass** die Umsetzung in Schritt a in einem Temperaturbereich von - 50 °C bis 100 °C erfolgt, die Umsetzung in Schritt c in einem Temperaturbereich von 0 °C bis 120 °C erfolgt, und wobei die Umsetzung in Schritt a bei einer Temperatur erfolgt, die um mindestens 15 °C niedriger ist als die Temperatur der Umsetzung in Schritt c.

16. Verfahren gemäß einem der Ansprüche 1 bis 15, **dadurch gekennzeichnet, dass** die Umsetzung in Schritt a in einem Druckbereich von 0,5 bis 10 bar erfolgt, die Umsetzung in Schritt c in einem Druckbereich von 1 bis 50 bar erfolgt, und wobei die Umsetzung in Schritt c bei einem Druck erfolgt, der um mindestens 0,1 bar höher ist als der Druck der Umsetzung in Schritt a.
